(19) Europäisches Patentamt
European Patent Office
Office européen des brevets

(11) EP 2 712 612 B1

# (12) EUROPEAN PATENT SPECIFICATION

(45) Date of publication and mention of the grant of the patent:
**25.03.2020 Bulletin 2020/13**

(51) Int Cl.:
***A61K 9/70*** *(2006.01)* ***A61K 31/40*** *(2006.01)*
***A61K 31/44*** *(2006.01)*

(21) Application number: **13186515.6**

(22) Date of filing: **27.09.2013**

(54) **Patch preparation containing amine oxide and isopropyl myristate**

Pflasterpräparat mir Aminoxid und Isopropylmyristat

Préparation pour patch contenant de l'oxyde d'amine et du myristate d'isopropyle

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2012 JP 2012217880**

(43) Date of publication of application:
**02.04.2014 Bulletin 2014/14**

(73) Proprietor: **NITTO DENKO CORPORATION**
**Osaka 567 (JP)**

(72) Inventors:
- **Nishimura, Masato**
**Ibaraki-shi, Osaka 567-8680 (JP)**
- **Inoue, Yoshitaka**
**Ibaraki-shi, Osaka 567-8680 (JP)**
- **Ito, Yoshiaki**
**Ibaraki-shi, Osaka 567-8680 (JP)**

(74) Representative: **J A Kemp LLP**
**14 South Square**
**Gray's Inn**
**London WC1R 5JJ (GB)**

(56) References cited:
**EP-A1- 2 759 294 WO-A2-2005/049090**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 712 612 B1

## Description

TECHNICAL FIELD OF THE INVENTION

The present invention relates to a patch preparation showing superior transdermal absorbability of a drug.

BACKGROUND OF THE INVENTION

Transdermal absorption preparation has many advantages. However, since many drugs show low transdermal absorbability, there are not many drugs actually formulated into transdermal absorption preparations, and a technique for improving transdermal absorbability of a drug has been desired.

Various methods have heretofore been considered to improve transdermal absorbability of a drug. For example, patent document 1 shows use of at least one kind of excipient (permeation enhancer or solubilizer) selected from amine oxide, unsaturated fatty acid, isopropyl myristate, lauroglycol, $\alpha$-terpineol, polyethylene glycol, sorbitan ester, lactic acid, dimethyl sulfoxide and combinations thereof, for improving transdermal absorbability of a drug, olanzapine.

Patent document 2 describes compositions for the transdermal administration of olanzapine. The compositions include olanzapine or a pharmaceutically acceptable salt thereof, a pressure sensitive adhesive, and an excipient, such as a permeation enhancer and/or a solubilizer of olanzapine. The compositions are said to be useful for the treatment of certain psychiatric disorders, for example schizophrenia and bipolar mania.

Patent document 3 describes a transdermal preparation comprising sequentially-stacked layers of a backing layer, a barrier layer, a drug adhesive layer and a release layer, wherein the drug adhesive layer contains a drug selected from the group consisting of fentanyl, an analogue thereof and a pharmaceutically-acceptable salt thereof, a skin permeation enhancer of the drug, and a polyacrylate adhesive, which shows a high skin permeation with a low drug dosage, equivalent to one with high drug dosage, by increasing the skin permeation rate of drug.

[Document List]

[patent document]

patent document 1: JP-A-2007-511605
patent document 2: WO 2005/049090
patent document 3: EP-A-2759294

SUMMARY OF THE INVENTION

Problems to be Solved by the Invention

The problem to be solved by the present invention is provision of a patch preparation showing superior transdermal absorbability of a drug.

Means of Solving the Problems

The present inventors have conducted intensive studies in an attempt to solve the above-mentioned problem, and found that transdermal absorbability of a drug can be markedly improved by using a combination of amine oxide and the below-mentioned low-polar liquid component. In addition, they have found that transdermal absorbability of a drug can be further improved by using the below-mentioned highly-polar liquid component together with the aforementioned combination. The present invention based on such findings is as described below.

[1] A patch preparation comprising a support and an adhesive layer on at least one surface of the support, wherein the adhesive layer comprises a drug, amine oxide, isopropyl myristate, and an adhesive base, and the adhesive layer has an amine oxide content of 0.1 - 5 wt% and an isopropyl myristate content of 30 - 50 wt%.

[2] The patch preparation of the aforementioned [1], wherein the adhesive base is acrylic polymer and/or a rubber-based polymer.

[3] The patch preparation of the aforementioned [1] or [2], wherein the amine oxide is dimethyllaurylamine oxide.

[4] The patch preparation of the aforementioned [1] - [3], wherein the adhesive layer further comprises a highly-polar liquid component based on at least one selected from the group consisting of diisopropyl sebacate, oleic acid, lauryl alcohol, isostearic acid, diethyl sebacate, propylene glycol monolaurate, diisopropyl adipate, sorbitan monooleate, sorbitan monostearate, sorbitan monopalmitate, propylene glycol monocaprylate, polyoxyethylene(2) lauryl ether, decanoic acid, polyoxyethylene(20) sorbitan trioleate, polyoxyethylene(20) sorbitan tristearate, sorbitan monolaurate, benzyl alcohol, polyoxyethylene(4.2)lauryl ether, valproic acid, octanoic acid, triacetine, propylene carbonate, triethyl citrate, polyoxyethylene(9) lauryl ether, 1-methyl-2-pyrrolidone, polyoxyethylene(20) sorbitan monooleate, polyoxyethylene(20) sorbitan monostearate, polyoxyethylene(20) monopalmitate, polyoxyethylene(20) monolaurate, dimethyl sulfoxide, dipropylene glycol, triisopropanolamine, levulinic acid, diethylene glycol monoethyl ether, diisopropanolamine, 1,3-butyleneglycol, monoisopropanolamine, triethanolamine, propylene glycol, diethanolamine, lactic acid, monoethanolamine, and glycerol, and has a highly-polar liquid component content of not more than 20 wt %.

[5] The patch preparation of the aforementioned [4], wherein the highly-polar liquid component is based on at least one selected from the group consisting of monoisopropanolamine, triethanolamine, propylene glycol, diethanolamine, lactic acid, monoethanolamine, and glycerol.

[6] The patch preparation of the aforementioned [5], wherein the highly-polar liquid component is based on lactic acid.

Effect of the Invention

According to the present invention, a patch preparation showing superior transdermal absorbability of a drug is obtained.

Description of Embodiments

The patch preparation of the present invention has an adhesive layer on at least one surface of a support, and the adhesive layer contains a drug, amine oxide, a low-polar liquid component and an adhesive base. In the following, each component of the adhesive layer is explained first, and then the support is explained.

[Amine oxide]

Amine oxide means a compound represented by the following formula (A) wherein $R^1$, $R^2$ and $R^3$ are each independently an organic group.

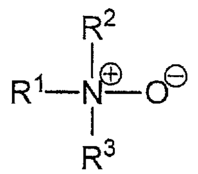

(A)

Only one kind of amine oxide may be used or two or more kinds thereof may be used in combination. As amine oxide, cyclic amine oxide wherein any two of $R^1$, $R^2$ and $R^3$ are bonded to form a ring (e.g., pyridine N-oxide, N-methylmorpholine N-oxide etc.) may be used. However, chain amine oxide not forming a ring is preferably used.

In chain amine oxide, $R^1$, $R^2$ and $R^3$ are preferably each independently a $C_{1-20}$ alkyl group optionally having substituent(s). The alkyl group may be a linear or branched chain, preferably linear. Examples of the substituent of the alkyl group include a halogen atom (e.g., fluorine atom, chlorine atom, bromine atom), a hydroxy group, an alkoxy group having 1 - 4 carbon atoms and the like, with preference given to a hydroxy group.

More preferably, in chain amine oxide, $R^1$ is an unsubstituted alkyl group having 10 - 20 carbon atoms, and $R^2$ and $R^3$ are each independently a $C_{1-8}$ alkyl group optionally having a hydroxy group. Further preferably, $R^1$ is an unsubstituted alkyl group having 10 - 20 carbon atoms, and $R^2$ and $R^3$ are each a $C_{1-4}$ alkyl group optionally having a hydroxy group.

Examples of chain amine oxide include dimethyllaurylamine oxide (trivial name: lauramine oxide, CAS No. 1643-20-5), bis(2-hydroxyethyl)laurylamine oxide (trivial name: dihydroxyethyl lauramine oxide, CAS No. 2530-44-1) and the like. Dimethyllaurylamine oxide is particularly preferable.

The content of amine oxide in the adhesive layer is 0.1 - 5 wt%, preferably 0.2 - 4.5 wt%, more preferably 0.5 - 4 wt%. When the content of amine oxide is less than 0.1 wt%, the drug may not show sufficiently high transdermal absorbability. On the other hand, when the content of amine oxide exceeds 5 wt%, skin irritation sometimes becomes high.

[Low-polar liquid component]

In the present invention, the low-polar liquid component is a liquid component based on an organic compound having an angle of 0 - 19° as calculated from the following formula (I): angle (°)=arctan (inorganic value/organic value) $\times$ (180/$\pi$) (I) and using an inorganic value and an organic value on an organic conceptual diagram (hereinafter sometimes to be abbreviated as an "angle on an organic conceptual diagram"). The variable of the inverse trigonometric function (i.e., arc tangent) of the above-mentioned formula (I) is radian, and (180/$\pi$) is a coefficient for converting the value of radian to the angle (°).

Here, the "component based on the organic compound" may be only one kind of organic compound, or a mixture of two or more kinds of compounds. In addition, the "liquid component" means a component having flowability at 32°C. The "component having flowability" is a component having a viscosity of not more than $1 \times 10^6$ mPa·s as measured with a cone-plate rotational viscometer at 0.5RPM and 32°C.

In the above-mentioned organic conceptual diagram, the properties of a compound are divided into organic value showing the level of covalent bond (hydrophobicity) and inorganic value showing the level of ionic bond (hydrophilicity), and an organic compound is plotted on orthogonal coordinates with the organic value on the x-axis (organic axis), and the inorganic value on the Y-axis (inorganic axis). The organic conceptual diagram is explained in detail in "New edition Organic Conceptual Diagram Foundation and Application" (YOSHIO KOUDA, YOSHIRO SATOU, YOSHIO HONMA, new edition, SANKYO PUBLISHING, November 30, 2008). In general, a compound closer to the x-axis (organic axis) shows higher hydrophobicity, and a compound closer to the Y-axis (inorganic axis) shows higher hydrophilicity.

In the present invention, the low-polar liquid component is isopropyl myristate.

The content of the low-polar liquid component in the adhesive layer is 30 - 50 wt%, preferably 30 - 45 wt%, more preferably 30 - 40 wt%. When the content of the low-polar liquid component is less than 30 wt%, the drug cannot show sufficiently high transdermal absorbability. On the other hand, when the content of the low-polar liquid component exceeds 50 wt%, the shape retention of the adhesive layer becomes low, which may cause extrusion of adhesive during preservation of the patch preparation and adhesive residue on the skin surface after detachment of the patch preparation therefrom.

The weight ratio of the low-polar liquid component and amine oxide in the adhesive layer (i.e., low-polar liquid component/amine oxide) is preferably 5 - 100, more preferably 10 - 80, further preferably 20 - 50. When the weight ratio is less than 5, skin irritation sometimes becomes high. On the other hand, when the weight ratio exceeds 100, the drug sometimes fails to show sufficiently high transdermal absorbability.

[Highly-polar liquid component]

The adhesive layer preferably contains a highly-polar liquid component. The highly-polar liquid component in the present invention is a liquid component based on an organic compound having an angle on an organic conceptual diagram of 20 - 80°. The organic compound having an angle on an organic conceptual diagram of 20 - 80° and having flowability at 32°C is directly used as a highly-polar liquid component. On the other hand, an organic compound having an angle on an organic conceptual diagram of 20 - 80°, which is solid at 32°C, can be used as a highly-polar liquid component by mixing the compound with an organic compound having an angle on an organic conceptual diagram of 20 - 80° and having flowability at 32°C and preparing the mixture as a liquid. In addition, an organic compound having an angle on an organic conceptual diagram of 20 - 80°, which is solid at 32°C, can be used as a highly-polar liquid component by preparing the compound as an aqueous solution. In such aqueous solution, the concentration of the organic compound in the aqueous solution needs to be not less than 80 wt%, preferably not less than 85 wt%, so that the property of the organic compound having an angle on an organic conceptual diagram of 20 - 80° can be sufficiently reflected in the aqueous solution.

As shown in the below-mentioned Examples, when a highly-polar liquid component is used in addition to the combination of amine oxide and low-polar liquid component, the transdermal absorbability of the drug can be synergistically improved. One kind or two or more kinds of the organic compound having an angle on an organic conceptual diagram of 20 - 80° may be used for the highly-polar liquid component.

The highly-polar liquid component is divided into

(i) the first highly-polar liquid component based on an organic compound having an angle on an organic conceptual diagram of 20 - 59°,
(ii) the second highly-polar liquid component based on an organic compound having an angle on an organic conceptual diagram of 60 - 69°, and
(iii) the third highly-polar liquid component based on an organic compound having an angle on an organic conceptual diagram of 70 - 80°.

Examples of the organic compound having an angle on an organic conceptual diagram of 20 - 59° to be used for the first highly-polar liquid component include diisopropyl sebacate (22°), oleic acid (23°), lauryl alcohol (23°), isostearic acid (23°), diethyl sebacate (23°), propylene glycol monolaurate (28°), diisopropyl adipate (29°), sorbitan monooleate (29°), sorbitan monostearate (31°), sorbitan monopalmitate (33°), propylene glycol monocaprylate (36°), polyoxyethylene(2) lauryl ether (36°), decanoic acid (37°), polyoxyethylene(20) sorbitan trioleate (38°), polyoxyethylene(20) sorbitan tristearate (39°), sorbitan monolaurate (39°), benzyl alcohol (39°), polyoxyethylene(4.2)lauryl ether (44°), valproic acid (45°), octanoic acid (43°), triacetine (45°), propylene carbonate (48°), triethyl citrate (49°), polyoxyethylene (9) lauryl ether (51°), 1-methyl-2-pyrrolidone (55°), polyoxyethylene(20) sorbitan monooleate (56°), polyoxyethylene(20) sorbitan monostearate (57°), polyoxyethylene(20) monopalmitate (58°), polyoxyethylene(20) monolaurate (59°) and the like. Only one kind of these may be used, or two or more kinds thereof may be used in combination.

Examples of the organic compound having an angle on an organic conceptual diagram of 60 - 69° to be used for the second highly-polar liquid component include dimethyl sulfoxide (60°), dipropylene glycol (61°), triisopropanolamine (64°), levulinic acid (65°), diethylene glycol monoethyl ether (66°), diisopropanolamine (66°), 1,3-butyleneglycol (68°) and the like. Only one kind of these may be used, or two or more kinds thereof may be used in combination.

Examples of the organic compound having an angle on an organic conceptual diagram of 70 - 80° to be used for the third highly-polar liquid component include monoisopropanolamine (71°), triethanolamine (72°), propylene glycol (73°), diethanolamine (73°), lactic acid (77°), monoethanolamine (77°), glycerol (79°) and the like. Only one kind of these may be used, or two or more kinds thereof may be used in combination.

From the aspect of transdermal absorbability of drugs, the highly-polar liquid component is preferably the second highly-polar liquid component and/or the third highly-polar liquid component, and the third highly-polar liquid component is more preferable.

When the content of the highly-polar liquid component in the adhesive layer is too high, the highly-polar liquid component is separated or blooms from the adhesive base, and a patch preparation having adhesiveness cannot be obtained. Therefore, the content of the highly-polar liquid component in the adhesive layer is limited to not more than 20 wt%. When a highly-polar liquid component is used, the content thereof in the adhesive layer is preferably 0.1 - 15 wt%, more preferably 1 - 10 wt%.

When a highly-polar liquid component is used, the weight ratio of the highly-polar liquid component and amine oxide in the adhesive layer (that is, highly-polar liquid component/amine oxide) is preferably 1 - 100, more preferably 2 - 50, further preferably 5 - 30. When the weight ratio is less than 1, skin irritation sometimes becomes high. On the other hand, when the weight ratio exceeds 100, the drug sometimes cannot show sufficiently high transdermal absorbability.

[Adhesive base]

The adhesive base means a polymer constituting a matrix of an adhesive layer. The adhesive base is mixed with the below-mentioned tackifier where necessary and constitutes an adhesive. Only one kind of the adhesive base may be used, or two or more kinds thereof may be used in combination. Examples of the adhesive base include acrylic polymers such as a (meth)acrylate-based polymer and the like; rubber-based polymers such as a styrene-isoprene-styrene block copolymer, a styrene-butadiene-styrene block copolymer, polyisoprene, polyisobutylene, polybutadiene and the like; silicone-based polymers such as a silicone rubber, a dimethylsiloxane base, a diphenylsiloxane base and the like; and the like. Among these, an acrylic polymer and a rubber-based polymer are preferable, and an acrylic polymer is more preferable.

Examples of the acrylic polymer include copolymers of

(i) alkyl (meth)acrylate (hereinafter sometimes to be abbreviated as "main monomer"),
(ii) a functional monomer having an unsaturated double bond and a functional group, and
(iii) other monomer as necessary. Only one kind of the main monomer, the functional monomer and other monomer may be used, or two or more kinds thereof may be used in combination.

In the aforementioned copolymer, the content of the main monomer is preferably 35 - 99.9 wt%, more preferably 50 - 98.9 wt%, the content of the functional monomer is preferably 0.1 - 15 wt%, more preferably 0.1 - 10 wt%, and the content of other monomer is preferably 0 - 50 wt%, more preferably 1 - 40 wt%.

The alkyl group of the main monomer (that is, alkyl (meth)acrylate) preferably has 4 - 13 carbon atoms. The alkyl group may be a linear or branched chain. Examples of the alkyl group include butyl, pentyl, hexyl, octyl, 2-ethylhexyl, nonyl, decyl, undecyl, dodecyl, tridecyl and the like. As the main monomer, 2-ethylhexyl acrylate and 2-ethylhexyl methacrylate are preferable, and 2-ethylhexyl acrylate is more preferable.

Examples of the functional group of the functional monomer include a carboxy group, a hydroxy group, an alkoxy group, an amino group, an amido group and the like. When two or more carboxy groups are present in the functional monomer, they may be bonded to each other to form an acid anhydride structure. Examples of the functional

monomer include carboxy group-containing monomers such as (meth)acrylic acid, itaconic acid, maleic acid, maleic anhydride and the like; hydroxy group-containing monomers such as hydroxyethyl (meth)acrylate, hydroxypropyl (meth)acrylate and the like; amino group-containing monomers such as aminoethyl (meth)acrylate, dimethylaminoethyl (meth)acrylate, tert-butylaminoethyl (meth)acrylate and the like; amido group-containing monomers such as (meth)acrylamide, dimethyl(meth)acrylamide, N-methylolpropane (meth)acrylamide, N-vinylacetamide and the like; alkoxy group-containing monomers such as methoxyethyl (meth)acrylate, ethoxyethyl (meth)acrylate, methoxyethyleneglycol (meth)acrylate, methoxydiethyleneglycol (meth)acrylate, methoxypolyethylene glycol (meth)acrylate, methoxypolypropyleneglycol (meth)acrylate and the like; and the like. Among these, a carboxy group-containing monomer is preferable, and (meth)acrylic acid is more preferable, from the aspects of pressure-sensitive adhesiveness and cohesiveness of the adhesive layer, releasability of a drug contained in the adhesive layer and the like.

Examples of other monomer include (meth)acrylonitrile, vinyl acetate, vinyl propionate, N-vinyl-2-pyrrolidone, methylvinylpyrrolidone, vinylpyrrolidone, vinylpiperidone, vinylpyrimidine, vinylpiperazine, vinylpyrrole, vinylimidazole, vinylcaprolactam, vinyloxazole and the like. Among these, N-vinyl-2-pyrrolidone is preferable.

As the acrylic polymer, a terpolymer of 2-ethylhexyl acrylate (main monomer), acrylic acid (functional monomer) and N-vinyl-2-pyrrolidone (other monomer) is preferable since it shows good adhesiveness to human skin, and adhesion and detaching can be easily repeated. The weight ratio of the terpolymer (that is, 2-ethylhexyl acrylate:acrylic acid:N-vinyl-2-pyrrolidone) is preferably 50 - 98.9:0.1 - 10:1 - 40.

While the glass transition temperature of the acrylic polymer varies depending on the composition of the copolymer, it is generally -100°C to -10°C, preferably -90°C to -20°C, in view of adhesiveness as a patch preparation. The glass transition temperature is a measured value of a differential scanning calorimeter. The absolute weight average molecular weight of the acrylic polymer (value of penetration chromatography/multi-angle laser light scattering detector) is preferably $1 \times 10^5$ - $6 \times 10^6$, more preferably $3 \times 10^5$ - $5 \times 10^6$, further preferably $1 \times 10^6$ - $4 \times 10^6$.

When an acrylic polymer is used as an adhesive base, it may be subjected to a crosslinking treatment by a suitable crosslinking method. By applying a crosslinking treatment, the adhesive layer becomes a gel state, which can suppress effusion of an adhesive layer component, and impart an appropriate cohesive force to the adhesive layer. The crosslinking treatment may be any of physical crosslinking treatments by radiation irradiation such as UV irradiation, electron beam irradiation and the like; chemical crosslinking treatments using a crosslinking agent such as polyisocyanate compound, organic peroxide, organic metal salt, metal alcoholate, metal chelate compound, polyfunctional compound and the like. When a crosslinking agent is used, while the amount thereof to be added varies depending on the kind of the crosslinking agent and the acrylic polymer, it is generally 0.1 - 2 parts by weight, preferably 0.15 - 1 part by weight, relative to 100 parts by weight of the acrylic polymer.

The viscosity average molecular weight of the rubber-based polymer is preferably 10,000 - 10,000,000, more preferably 40,000 - 5,000,000. The viscosity average molecular weight is obtained by calculating the Staudinger index ($J_0$) according to the Schulz-Blaschke equation from the flow time of capillary of the Ubbelohde's viscometer at 20°C, and applying the $J_0$ value to the following equations.

[formula 1]

$$J_0=\eta_{sp}/\{c(1+0.31\eta_{sp})\} \quad \text{(Schulz-Blaschke equation)}$$

$$\eta_{sp}=t/t_0-1$$

t: flow time of solution (according to Hagenbach-couette correction)
$t_0$: flow time of solvent (according to Hagenbach-couette correction)
c: concentration of solution ($g/cm^3$)

$$J_0=3.06 \times 10^{-2} Mv^{0.65}$$

Mv: viscosity average molecular weight

The rubber-based polymer preferably contains at least one kind selected from polyisobutylene, polyisoprene and styrene-diene-styrene block copolymer as a main component, more preferably contains at least one kind selected from polyisobutylene, polyisoprene, styrene-butadiene-styrene block copolymer (SBS) and styrene-isoprene-styrene block copolymer (SIS) as a main component. Here, the "main component" means that the total amount of the aforementioned at least one kind is not less than 50 wt% of the total of the rubber-based polymer and the tackifier. A blend of a rubber-based polymer containing a high molecular weight polyisobutylene having a viscosity average molecular weight of 3,000,000 - 5,000,000 and a low molecular weight polyisobutylene having a viscosity average molecular weight of 40,000 - 85,000 at a weight ratio of 95:5 - 5:95 is more preferable, since drug stability is high, and the necessary adhesive force and the cohesive force can be simultaneously achieved.

The content of the adhesive base in the adhesive layer is 5 - 65 wt%, preferably 10 - 65 wt%, more preferably 15 - 60 wt%. When the content of the adhesive base is less than 5 wt%, the internal cohesive force of the adhesive layer may decrease. On the other hand, when the content of the adhesive base exceeds 65 wt%, the adhesive layer becomes hard and the tackiness sometimes decreases.

[Drug]

The drug to be contained in the patch preparation of the present invention is not particularly limited as long as it can be administered via the skin of a mammal such as human and the like, i.e., transdermally absorbable drug. Specific examples of such drug include general anesthetics, hypnotic sedatives, antiepileptic drugs, antipyretic analgesic antiphlogistic drugs, anti-vertiginous drugs, psychoneurotic drugs, central neurological drug, antidementia, topical anesthetics, skeletal muscle relaxants, autonomic drugs, antispasmodic drugs, anti-parkinsonian drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for purulent diseases, analgesic-antipruritic-styptic-antiinflammatory drugs, drugs for parasitic skin diseases, hemostatic drugs, gout treatment drugs, drugs for diabetes, anti-malignant tumor agents, antibiotic, chemical therapy agents, narcotic, quit smoking aids and the like.

The drug may also take the form of a physiologically acceptable salt. While such salt is not particularly limited, for example, addition salts with organic acids such as formate, acetate, lactate, adipate, citrate, tartrate, methanesulfonate (also referred to as mesilate), benzenesulfonate (also referred to as besilate), fumarate, maleate and the like; addition salts with inorganic acids such as hydrochloride, sulfate, nitrate salt, phosphate and the like; addition salts with organic bases such as meglumine salt, piperazine salt, tromethamine salt, choline salt, diethylamine salt, tert-butylamine salt and the like; addition salts with inorganic bases such as sodium salt, calcium salt, potassium salt, magnesium salt, aluminum salt, ammonium salt and the like can be mentioned. The drug may be a solvate (for example, hydrate, ethanol solvate, propylene glycol solvate) or nonsolvate.

As the drug, any of a liquid drug having flowability at 32°C and a solid drug that takes the form of a solid at 32°C can be used. Here, the solid drug generally has low transdermal absorbability than liquid drugs. However, the patch preparation of the present invention containing such solid drug can achieve superior transdermal absorbability.

The content of the drug in the adhesive layer is not particularly limited as long as it can exhibit the effect of the drug and does not impair the adhesiveness of the adhesive layer. For example, it is 0.1 - 60 wt%, preferably 0.5 - 40 wt%. When the content of the drug is less than 0.1 wt%, the treatment effect may not be sufficient and, when it exceeds 60 wt%, the adhesive layer may not show sufficient adhesiveness.

[Other component]

The adhesive layer may contain a component other than a drug, amine oxide, a low-polar liquid component and a highly-polar liquid component. Examples of other component include organic powder, inorganic powder, tackifier, polyvinylpyrrolidone, natural polysaccharides and a derivative thereof, cyclodextrin, aminoalkyl methacrylate copolymer, cellulose derivative (methylcellulose, ethylcellulose, hydroxypropylcellulose) and the like. Only one kind of other component may be used, or two or more kinds thereof may be used in combination.

Examples of the organic powder include crosslinked polyvinylpyrrolidone, crystalline cellulose, polyethylene and the like. Among these, crosslinked polyvinylpyrrolidone is preferable. Examples of the inorganic powder include aluminum silicate, magnesium silicate, calcium silicate, magnesium aluminum silicate, magnesium aluminosilicate, magnesium aluminometasilicate, aluminum oxide, zinc oxide, calcium oxide, titanium oxide, magnesium oxide, aluminum stearate, zinc stearate, potassium stearate, sodium stearate, magnesium stearate and the like.

When a rubber-based polymer is used as an adhesive base and the adhesive layer contains a highly-polar liquid component, a higher polarity of the highly-polar liquid component (i.e., higher angle, on an organic conceptual diagram, of the organic compound used for a highly-polar liquid component) tends to cause separation and blooming of the highly-polar liquid component. This tendency can be suppressed by adding a powder to the adhesive layer. In this case, the powder is preferably an organic powder.

The average particle size of the powder is preferably 0.01 - 200 $\mu$m, more preferably 0.02 - 100 $\mu$m. The average particle size can be measured by a laser diffraction particle size analyzer.

When a powder is used, the content thereof in an adhesive layer is preferably 0.1 - 30 wt%, more preferably

0.1 - 20 wt%. When the content of the powder is less than 0.1 wt%, the suppressive effect on the separation and blooming of a highly-polar organic component may not be sufficient. When it exceeds 30 wt%, the adhesive force decreases and the adhesiveness may be poor.

When the adhesive base has insufficient adhesiveness at ambient temperature, it is preferable to further add a tackifier to the adhesive layer to confer adhesiveness at ambient temperature. When a rubber-based polymer is used as an adhesive base, and even when an acrylic polymer is used as an adhesive base, a tackifier may be further added to potentiate the adhesiveness of the adhesive layer. As a tackifier, one known in the field of patch preparations can be appropriately selected and used. Examples thereof include petroleum resin (e.g., aromatic petroleum resin, aliphatic petroleum resin and the like), terpene resin, rosin resin, coumarone indene resin, styrene resin (e.g., styrene resin, poly($\alpha$-methylstyrene) and the like), hydrogenated petroleum resin (e.g., alicyclic saturated hydrocarbon resin and the like) and the like. Among these, an alicyclic saturated hydrocarbon resin is preferable since preservation stability of a drug is improved. When a tackifier is used, the content thereof in an adhesive layer is generally not less than 30 parts by weight and less than 100 parts by weight, preferably not less than 50 parts by weight and less than 100 parts by weight, per 100 parts by weight of the adhesive base.

[Support]

In the present invention, the support is not particularly limited, and a support used in the field of patch preparation can be used. Examples of the material of the support include polyester (e.g., poly(ethylene terephthalate) (PET) etc.), nylon (registered trade mark), saran (registered trade mark), polyvinyl chloride, polyethylene, polypropylene, ethylene-vinyl acetate copolymer, polytetrafluoroethylene, ionomer resin and the like; metal; and the like. The support may be a single layer of the aforementioned materials, or a laminate thereof. To improve the adhesiveness (anchor property) between a support and an adhesive layer, a laminate film of a non-porous film made from the aforementioned materials and the below-mentioned porous film is preferably used as a support, and an adhesive layer is preferably formed on the porous film side. Examples of the porous film include paper, woven fabric, non-woven fabric and the like. Moreover, the aforementioned non-porous film subjected to a mechanical perforation treatment can be used as a porous film. As a porous film, paper, woven fabric, non-woven fabric (e.g., polyester non-woven fabric, and poly(ethylene terephthalate)non-woven fabric etc.) are preferable from the aspects of flexibility.

While the thickness of the support is not particularly limited, it is preferably 2 - 200 $\mu$m, more preferably 10 - 50 $\mu$m. When a laminate film of a non-porous film and a porous film is used as a support, the thickness of the non-porous film is preferably 2 - 100 $\mu$m, more preferably 2 - 50 $\mu$m, and a weight per unit area of the porous film (e.g., paper, woven fabric, non-woven fabric) is preferably 5 - 30 g/$m^2$ from the aspect of anchor property.

[Release liner]

The patch preparation of the present invention preferably has a release liner laminated on the adhesive face of the adhesive layer until use so that the adhesive face can be protected. A release liner permitting a release treatment and having a sufficiently light release force can be used. For example, a release liner obtained by subjecting a substrate made of a film such as polyester (e.g., poly(ethylene terephthalate) etc.), polyvinyl chloride, polyvinyldene chloride, and the like; paper such as fine paper, glassine paper and the like; or a laminate film of the above-mentioned film and paper; and the like, to a release treatment can be used. Examples of the release treatment include application of a silicone resin, fluororesin and the like. From the aspects of barrier property and cost, a release liner obtained by a release treatment of polyester (particularly, poly(ethylene terephthalate)) is preferable. The thickness of the release liner is generally 10 - 200 $\mu$m, preferably 25 - 100 $\mu$m. Examples

The present invention is explained in detail in the following by referring to Examples, which are not to be construed as limitative. Unless otherwise specified, the following "parts" means "parts by weight".

The starting materials used in the Examples and Comparative Examples are as described below.

(1) drug

(i) perindopril erbumine
(ii) amlodipine besilate

(2) amine oxide
lauramine oxide
(3) low-polar liquid component
isopropyl myristate (angle on an organic conceptual diagram 10°)
(4) first highly-polar liquid component

oleic acid (angle on an organic conceptual diagram 23°)
(5) second highly-polar liquid component

(i) dimethyl sulfoxide (angle on an organic conceptual diagram 60°)
(ii) dipropylene glycol (angle on an organic conceptual diagram 61°)

(6) third highly-polar liquid component
90 wt% aqueous solution of lactic acid (angle on an organic conceptual diagram 77°)
(7) other component

(i) organic powder: crosslinked polyvinylpyrrolidone (average particle size 7 μm, "Kollidon CL-M" manufactured by BASF)
(ii) tackifier: alicyclic saturated hydrocarbon resin ("ARKON P100" manufactured by Arakawa Chemical Industries, Ltd.)

(8) adhesive base

(i) acrylic polymer
Under an inert gas atmosphere, acrylic acid 2-ethylhexyl (75 parts), N-vinyl-2-pyrrolidone (22 parts), acrylic acid (3 parts) and azobisisobutyronitrile (0.2 parts) were subjected to solution polymerization in ethyl acetate (60°C) to give solution A of an acrylic copolymer (solid content: 28 wt%) (weight average absolute molecular weight: 1,900,000).
(ii) rubber-based polymer
High molecular weight polyisobutylene having a viscosity average molecular weight of 4,000,000 (24 parts), low molecular weight polyisobutylene having a viscosity average molecular weight of 55,000 (36 parts), and a tackifier (40 parts) were mixed to give mixture B containing a rubber-based polymer.

[Examples 1 - 5 and Comparative Examples 1 - 11]

Acrylic copolymer solution A, perindopril erbumine, amine oxide, a low-polar liquid component and a highly-polar liquid component were mixed to achieve the adhesive layer compositions described in Table 1, whereby ethyl acetate solutions for forming adhesive layers were prepared. Dimethyl sulfoxide was used as the second highly-polar liquid component in Comparative Example 6, and dipropylene glycol was used in other Examples and Comparative Examples. The obtained solutions were applied to a poly(ethylene terephthalate)(PET) liner (thickness 75 μm) subjected to a silicone release treatment such that the dry thickness of the adhesive layer was about 60 μm, and dried to form an adhesive layer. The adhesive layer was laminated on a PET support to give a patch preparation in a sheet. To examine transdermal absorbability of the drug, the obtained patch preparation was subjected to a skin permeability test.

[Examples 6 - 8 and Comparative Examples 12 - 14]

Mixture B containing a rubber-based polymer, perindopril erbumine, amine oxide, a low-polar liquid component, a highly-polar liquid component, an organic powder and a mixed solvent of toluene/isopropanol were mixed to achieve the adhesive layer compositions described in Table 2, whereby toluene/isopropanol solutions for forming adhesive layers were prepared. Dipropylene glycol was used as the second highly-polar liquid component. The obtained solutions were applied to a poly(ethylene terephthalate)(PET) liner (thickness 75 μm) subjected to a silicone release treatment such that the dry thickness of the adhesive layer was about 60 μm, and dried to form an adhesive layer. The adhesive layer was laminated on a PET support to give a patch preparation in a sheet. To examine transdermal absorbability of the drug, the obtained patch preparation was subjected to a skin permeability test.

[Examples 9 - 13 and Comparative Examples 15 - 23]

Acrylic copolymer solution A, amlodipine besilate, amine oxide, a low-polar liquid component and a highly-polar liquid component were mixed to achieve the adhesive layer compositions described in Table 3, whereby ethyl acetate solutions for forming adhesive layers were prepared. Dipropylene glycol was used as the second highly-polar liquid component. The obtained solutions were applied to a poly(ethylene terephthalate)(PET) liner (thickness 75 μm) subjected to a silicone release treatment such that the dry thickness of the adhesive layer was about 60 μm, and dried to form an adhesive layer. The adhesive layer was laminated on a PET support to give a patch preparation in a sheet. To examine transdermal absorbability of the drug, the obtained patch preparation was subjected to a skin permeability test.

[Examples 14 - 16 and Comparative Examples 24 - 26]

Mixture B containing a rubber-based polymer, amlodipine besilate, amine oxide, a low-polar liquid component, a highly-polar liquid component, an organic powder and a mixed solvent of toluene/isopropanol were mixed to achieve the adhesive layer compositions described in Table 4, whereby toluene/isopropanol solutions for forming adhesive layers were prepared. Dipropylene glycol was used as the second highly-polar liquid component. The obtained solutions were applied to a poly (ethylene terephthalate)(PET) liner (thickness 75 μm) subjected to a silicone release treatment such that the dry thickness of the adhesive layer was-about 60 μm, and dried to form an adhesive layer. The adhesive layer was laminated on a PET support to give a patch preparation in a sheet. To examine transdermal absorbability of the drug, the obtained patch preparation was subjected to a skin permeability test.

[skin permeability test]

A patch preparation was cut into a circle having a diameter of 6 mmϕ, adhered to the stratum corneum side of a skin isolated from a hairless mouse, mounted on a cell for skin permeation experiment (effective area 9 mmϕ) such that the dermic layer side was a receptor phase, and a skin permeation experiment was conducted. As a receptor solution, a deaerated PBS(-) solution (phosphate buffered saline) was used. The receptor solution was sampled over time and the concentration of the permeated drug was quantified by HPLC (high performance liquid chromatography). The skin permeation experiment was performed for 12 hr for the patch preparations of Examples 1 - 8 and Comparative Examples 1 - 14 using perindopril erbumine as a drug, and 24 hr for the patch preparations of Examples 9 - 16 and Comparative Example 15 - 26 using amlodipine besilate as a drug. Average values (N=2) of a cumulative permeation amount for 12 hr or 24 hr are shown in the following Tables 1 - 4. In the following Tables 3 and 4, cumulative permeation amounts of amlodipine for 24 hr, not that of amlodipine besilate, are shown.

Table 1

| | drug*1 (parts) | acrylic polymer (parts) | amine oxide (parts) | low-polar liquid component (parts) | first highly-polar liquid component (parts) | second highly-polar liquid*2 component (parts) | third highly-polar liquid component (parts) | 12 hr cumulative permeation amount *3 ($\mu g/cm^2$) |
|---|---|---|---|---|---|---|---|---|
| Comp. Ex. 1 | 5 | 75 | - | 20 | - | - | - | 6 |
| Comp. Ex. 2 | 5 | 70 | - | 20 | - | 5 | - | 10 |
| Comp. Ex. 3 | 5 | 74 | 1 | 20 | - | - | - | 3 |
| Comp. Ex. 4 | 5 | 69 | 1 | 20 | - | 5 | - | 22 |
| Comp. Ex. 5 | 5 | 59 | 1 | - | 35 | - | - | 1 |
| Comp. Ex. 6 | 5 | 59 | - | 7 | 7 | 22 | - | 2 |
| Comp. Ex. 7 | 5 | 65 | - | 30 | - | - | - | 23 |
| Comp. Ex. 8 | 5 | 60 | - | 30 | - | 5 | - | 36 |
| Comp. Ex. 9 | 5 | 60 | - | 30 | - | - | 5 | 38 |
| Comp. Ex. 10 | 5 | 55 | - | 40 | - | - | - | 77 |
| Comp. Ex. 11 | 5 | 50 | - | 40 | - | 5 | - | 90 |
| Ex. 1 | 5 | 64 | 1 | 30 | - | - | - | 31 |
| Ex. 2 | 5 | 59 | 1 | 30 | - | 5 | - | 61 |
| Ex. 3 | 5 | 59 | 1 | 30 | - | - | 5 | 149 |
| Ex. 4 | 5 | 54 | 1 | 40 | - | - | - | 107 |

(continued)

| | drug*1 (parts) | acrylic polymer (parts) | amine oxide (parts) | low-polar liquid component (parts) | first highly-polar liquid component (parts) | second highly-polar liquid*2 component (parts) | third highly-polar liquid component (parts) | 12 hr cumulative permeation amount *3 ($\mu g/cm^2$) |
|---|---|---|---|---|---|---|---|---|
| Ex. 5 | 5 | 49 | 1 | 40 | - | 5 | - | 133 |

*1 Perindopril erbumine was used as a drug.
*2 As the second highly-polar liquid component, dimethyl sulfoxide was used in Comparative Example 6 and dipropylene glycol was used in other Examples.
*3 shows permeation amount of perindopril erbumine.

Table 2

| | drug *1 (parts) | rubber-based polymer (parts) | tackifier (parts) | amine oxide (parts) | low-polar liquid component (parts) | second highly-polar liquid component *2 (parts) | third highly-polar liquid component (parts) | organic powder (parts) | 12 hr cumulative permeation amount *3 ($\mu g/cm^2$) |
|---|---|---|---|---|---|---|---|---|---|
| Comp. Ex. 12 | 5 | 39 | 26 | - | 30 | - | - | - | 31 |
| Comp. Ex. 13 | 5 | 36 | 24 | - | 30 | 5 | - | - | 22 |
| Comp. Ex. 14 | 5 | 30 | 20 | - | 30 | - | 5 | 10 | 37 |
| Ex. 6 | 5 | 38.4 | 25.6 | 1 | 30 | - | - | - | 59 |
| Ex. 7 | 5 | 35.4 | 23.6 | 1 | 30 | 5 | - | - | 72 |
| Ex. 8 | 5 | 29.4 | 19.6 | 1 | 30 | - | 5 | 10 | 234 |

*1 Perindopril erbumine was used as a drug.
*2 As the second highly-polar liquid component, dipropylene glycol was used.
*3 shows permeation amount of perindopril erbumine.

Table 3

| | drug *1 (parts) | acrylic polymer (parts) | amine oxide (parts) | low-polar liquid component (parts) | second highly-polar liquid component*2 (parts) | third highly-polar liquid component (parts) | 24 hr cumulative permeation amount*3 ($\mu g/cm^2$) |
|---|---|---|---|---|---|---|---|
| Comp. Ex. 15 | 5 | 75 | - | 20 | - | - | 0 |
| Comp. Ex. 16 | 5 | 70 | - | 20 | 5 | - | 0 |
| Comp. Ex. 17 | 5 | 65 | - | 30 | - | - | 0 |
| Comp. Ex. 18 | 5 | 60 | - | 30 | 5 | - | 0 |
| Comp. Ex. 19 | 5 | 60 | - | 30 | - | 5 | 0 |
| Comp. Ex. 20 | 5 | 55 | - | 40 | - | - | 0 |
| Comp. Ex. 21 | 5 | 50 | - | 40 | 5 | - | 0 |
| Comp. Ex. 22 | 5 | 70 | 5 | 20 | - | - | 0 |
| Comp. Ex. 23 | 5 | 65 | 5 | 20 | 5 | - | 0 |
| Ex. 9 | 5 | 60 | 5 | 30 | - | - | 1 |
| Ex. 10 | 5 | 55 | 5 | 30 | 5 | - | 3 |
| Ex. 11 | 5 | 55 | 5 | 30 | - | 5 | 10 |
| Ex. 12 | 5 | 50 | 5 | 40 | - | - | 4 |
| Ex. 13 | 5 | 45 | 5 | 40 | 5 | - | 5 |

*1 Amlodipine besilate was used as a drug.
*2 As the second highly-polar liquid component, dipropylene glycol was used.
*3 shows permeation amount of amlodipine.

Table 4

| | drug *1 (parts) | rubber-based polymer (parts) | tackifier (parts) | amine oxide (parts) | low-polar liquid component (parts) | second highly-polar liquid component *2 (parts) | third highly-polar liquid component (parts) | organic powder (parts) | 24 hr cumulative permeation amount*3 ($\mu g/cm^2$) |
|---|---|---|---|---|---|---|---|---|---|
| Comp. Ex. 24 | 5 | 39 | 26 | - | 30 | - | - | - | 0 |
| Comp. Ex. 25 | 5 | 36 | 24 | - | 30 | 5 | - | - | 0 |
| Comp. Ex. 26 | 5 | 30 | 20 | - | 30 | - | 5 | 10 | 0 |
| Ex. 14 | 5 | 36 | 24 | 5 | 30 | - | - | - | 23 |
| Ex. 15 | 5 | 33 | 22 | 5 | 30 | 5 | - | - | 20 |
| Ex. 16 | 5 | 27 | 18 | 5 | 30 | - | 5 | 10 | 44 |

*1 Amlodipine besilate was used as a drug.
*2 As the second highly-polar liquid component, dipropylene glycol was used.
*3 shows permeation amount of amlodipine.

As is clear from the above-mentioned Table 1, in Examples 1 - 3 wherein 30 parts of the low-polar liquid component was used, the permeation amount exceeded that of Comparative Examples 7 - 9 wherein an equivalent amount of the low-polar liquid component was used. This shows an effect provided by the use of appropriate amounts of amine oxide and the low-polar liquid component. In Example 2, the permeation amount exceeded the arithmetical mean value of the permeation amount in Comparative Example 8 and Example 1. This shows a synergistic effect provided by the combined use of amine oxide, a low-polar liquid component and a highly-polar liquid component. In Example 3, the permeation amount exceeded the arithmetical mean value of the permeation amount in Comparative Example 9 and Example 1. This shows a synergistic effect provided by the combined use of amine oxide, a low-polar liquid component and a highly-polar liquid component. Particularly, the permeation amount was higher in Example 3 than in Example 2.
As is clear from the above-mentioned Table 1, the permeation amount in Examples 4 and 5 wherein 40 parts of the low-polar liquid component was used also exceeded that in Comparative Examples 10 and 11 wherein an equivalent amount of the low-polar liquid component was used.
As is clear from the above-mentioned Table 2, in Examples 6 - 8, the permeation amount exceeded that of Comparative Examples 12 - 14. This shows an effect provided by the use of appropriate amounts of amine oxide and the low-polar liquid component. In Example 7, the permeation amount exceeded the arithmetical mean value of the permeation amount in Comparative Example 13 and Example 6. This shows a synergistic effect provided by the combined use of amine oxide, a low-polar liquid component and a highly-polar liquid component. In Example 8, the permeation amount exceeded the arithmetical mean value of the permeation amount in Comparative Example 14 and Example 6. This shows a synergistic effect provided by the combined use of amine oxide, a low-polar liquid component and a highly-polar liquid component. Particularly, the permeation amount was higher in Example 8 than in Example 7.
As is clear from the above-mentioned Tables 3 and 4, when amlodipine besilate showing low skin permeability is used as a drug (the above-mentioned Tables 3 and 4), the skin permeability of the drug can be improved by using appropriate amounts of amine oxide and the low-polar liquid component, as in the case of perindopril erbumine (the above-mentioned Tables 1 and 2).

[Comparative Examples 27 and 28]

An ethyl acetate solution for forming an adhesive layer was prepared to achieve an adhesive layer composition of perindopril erbumine (5 parts), acrylic polymer (64 parts), lauramine oxide (1 part) and dipropylene glycol (30 parts). The obtained solutions were applied to a poly(ethylene terephthalate)(PET) liner (thickness 75 μm) subjected to a silicone release treatment such that the dry thickness of the adhesive layer was about 60 μm, and dried to form an adhesive layer. The adhesive layer was laminated on a PET support to give the patch preparation of Comparative Example 27.
In the same manner as in Comparative Example 27 except that an aqueous lactic acid solution was used instead of dipropylene glycol, the patch preparation of Comparative Example 28 was obtained.
In the patch preparations obtained in Comparative Examples 27 and 28, adhesiveness could not be obtained since a highly-polar liquid component (dipropylene glycol or aqueous lactic acid solution) was separated or bloomed from the adhesive base (acrylic polymer).

[Comparative Examples 29 and 30]

A toluene/isopropanol solution for forming an adhesive layer was prepared to achieve an adhesive layer composition of perindopril erbumine- (5 parts), a rubber-based polymer (32.4 parts), a tackifier (21.6 parts), lauramine oxide (1 part) and dipropylene glycol (30parts), and an organic powder (10 parts). The obtained solutions were applied to a poly(ethylene terephthalate)(PET) liner (thickness 75 μm) subjected to a silicone release treatment such that the dry thickness of the adhesive layer was about 60 μm, and dried to form an adhesive layer. The adhesive layer was laminated on a PET support to give the patch preparation of Comparative Example 29.
In the same manner as in Comparative Example 29 except that an aqueous lactic acid solution was used instead of dipropylene glycol, the patch preparation of Comparative Example 30 was obtained.
In the patch preparations obtained in Comparative Examples 29 and 30, adhesiveness could not be obtained since a highly-polar liquid component (dipropylene glycol or aqueous lactic acid solution) was separated or bloomed from the adhesive base (rubber-based polymer).

## Claims

1. A patch preparation comprising a support and an adhesive layer on at least one surface of the support, wherein

   the adhesive layer comprises

a drug,
amine oxide,
isopropyl myristate, and
an adhesive base, and
the adhesive layer has an amine oxide content of 0.1 - 5 wt% and an isopropyl myristate content of 30 - 50 wt%.

2. The patch preparation according to claim 1, wherein the adhesive base is acrylic polymer and/or a rubber-based polymer.

3. The patch preparation according to claim 1 or 2, wherein the amine oxide is dimethyllaurylamine oxide.

4. The patch preparation according to any one of claims 1 - 3, wherein the adhesive layer further comprises a highly-polar liquid component based on at least one selected from the group consisting of diisopropyl sebacate, oleic acid, lauryl alcohol, isostearic acid, diethyl sebacate, propylene glycol monolaurate, diisopropyl adipate, sorbitan monooleate, sorbitan monostearate, sorbitan monopalmitate, propylene glycol monocaprylate, polyoxyethylene(2) lauryl ether, decanoic acid, polyoxyethylene(20) sorbitan trioleate, polyoxyethylene(20) sorbitan tristearate, sorbitan monolaurate, benzyl alcohol, polyoxyethylene(4.2)lauryl ether, valproic acid, octanoic acid, triacetine, propylene carbonate, triethyl citrate, polyoxyethylene(9) lauryl ether, 1-methyl-2-pyrrolidone, polyoxyethylene(20) sorbitan monooleate, polyoxyethylene(20) sorbitan monostearate, polyoxyethylene(20) monopalmitate, polyoxyethylene(20) monolaurate, dimethyl sulfoxide, dipropylene glycol, triisopropanolamine, levulinic acid, diethylene glycol monoethyl ether, diisopropanolamine, 1,3-butyleneglycol, monoisopropanolamine, triethanolamine, propylene glycol, diethanolamine, lactic acid, monoethanolamine, and glycerol, and has a highly-polar liquid component content of not more than 20 wt%.

5. The patch preparation according to claim 4, wherein the highly-polar liquid component is based on at least one selected from the group consisting of monoisopropanolamine, triethanolamine, propylene glycol, diethanolamine, lactic acid, monoethanolamine, and glycerol.

6. The patch preparation according to claim 5, wherein the highly-polar liquid component is based on lactic acid.

## Patentansprüche

1. Pflasterpräparat, umfassend einen Träger und eine Kleberschicht auf wenigstens einer Fläche des Trägers, wobei die Kleberschicht einen Wirkstoff, Aminoxid, Isopropylmyristat und eine Kleberbasis umfasst und
die Kleberschicht einen Aminoxidgehalt von 0,1 bis 5 Gew.-% und einen Isopropylmyristatgehalt von 30 bis 50 Gew.-% aufweist.

2. Pflasterpräparat gemäß Anspruch 1, wobei es sich bei der Kleberbasis um ein Acrylpolymer und/oder ein Polymer auf Kautschukbasis handelt.

3. Pflasterpräparat gemäß Anspruch 1 oder 2, wobei es sich bei dem Aminoxid um Dimethyllaurylaminoxid handelt.

4. Pflasterpräparat gemäß einem der Ansprüche 1 bis 3, wobei die Kleberschicht weiterhin eine hochgradig polare flüssige Komponente umfasst, die auf wenigstens einer beruht, die aus der Gruppe, die aus Diisopropylsebacat, Ölsäure, Laurylalkohol, Isostearinsäure, Diethylsebacat, Propylenglycolmonolaurat, Diisopropyladipat, Sorbitanmonooleat, Sorbitanmonostearat, Sorbitanmonopalmitat, Propylenglycolmonocaprylat, Polyoxyethylen(2)laurylether, Decansäure, Polyoxyethylen(20)sorbitantrioleat, Polyoxyethylen(20)sorbitantristearat, Sorbitanmonolaurat, Benzylalkohol, Polyoxyethylen(4.2)laurylether, Valprosäure, Octansäure, Triacetin, Propylencarbonat, Triethylcitrat, Polyoxyethylen(9)laurylether, 1-Methyl-2-pyrrolidon, Polyoxyethylen(20)sorbitanmonooleat, Polyoxyethylen(20)sorbitanmonostearat, Polyoxyethylen(20)monopalmitat, Polyoxyethylen(20)monolaurat, Dimethylsulfoxid, Dipropylenglycol, Triisopropanolamin, Lävulinsäure, Diethylenglycolmonoethylether, Diisopropanolamin, 1,3-Butylenglycol, Monoisopropanolamin, Triethanolamin, Propylenglycol, Diethanolamin, Milchsäure, Monoethanolamin und Glycerin besteht, ausgewählt ist und einen Gehalt an der hochgradig polaren flüssigen Komponente von nicht mehr als 20 Gew.-% aufweist.

5. Pflasterpräparat gemäß Anspruch 4, wobei die hochgradig polare flüssige Komponente auf wenigstens einer beruht, die aus der Gruppe ausgewählt ist, die aus Monoisopropanolamin, Triethanolamin, Propylenglycol, Diethanolamin,

Milchsäure, Monoethanolamin und Glycerin besteht.

6. Pflasterpräparat gemäß Anspruch 5, wobei die hochgradig polare flüssige Komponente auf Milchsäure beruht.

**Revendications**

1. Préparation de patch comprenant un support et une couche adhésive sur au moins une surface du support, dans laquelle
la couche adhésive comprend
un médicament,
un oxyde d'amine,
du myristate d'isopropyle, et
une base adhésive, et
la couche adhésive a une teneur en oxyde d'amine de 0,1 à 5 % en poids et une teneur en myristate d'isopropyle de 30 à 50 % en poids.

2. Préparation de patch selon la revendication 1, dans laquelle la base adhésive est un polymère acrylique et/ou un polymère à base de caoutchouc.

3. Préparation de patch selon la revendication 1 ou 2, dans laquelle l'oxyde d'amine est l'oxyde de diméthyl-laurylamine.

4. Préparation de patch selon l'une quelconque des revendications 1 à 3, dans laquelle la couche adhésive comprend en outre un composant liquide fortement polaire à base de l'au moins un choisi dans le groupe constitué par le sébaçate de diisopropyle, l'acide oléique, l'alcool laurylique, l'acide isostéarique, le sébaçate de diéthyle, le monolaurate de propylèneglycol, l'adipate de diisopropyle, le monooléate de sorbitan, le monostéarate de sorbitan, le monopalmitate de sorbitan, le monocaprylate de propylèneglycol, le lauryléther polyoxyéthyléné(2), l'acide décanoïque, le trioléate de sorbitan polyoxyéthyléné(20), le tristéarate de sorbitan polyoxyéthyléné(20), le monoolaurate de sorbitan, l'alcool benzylique, le lauryléther polyoxyéthyléné(4.2), l'acide valproïque, l'acide octanoïque, la triacétine, le carbonate de propylène, le citrate de triéthyle, le lauryléther polyoxyéthyléné(9), la 1-méthyl-2-pyrrolidone, le monooléate de sorbitan polyoxyéthyléné(20), le monostéarate de sorbitan polyoxyéthyléné(20), le monopalmitate polyoxyéthyléné(20), le monolaurate polyoxyéthyléné(20), le diméthylsulfoxyde, le dipropylèneglycol, la triisopropanolamine, l'acide lévulinique, l'éther monoéthylique de diéthylèneglycol, la diisopropanolamine, le 1,3-butylèneglycol, la monoisopropanolamine, la triéthanolamine, le propylèneglycol, la diéthanolamine, l'acide lactique, la monoéthanolamine, et le glycérol, et a une teneur en composant liquide fortement polaire non supérieure à 20 % en poids.

5. Préparation de patch selon la revendication 4, dans laquelle le composant liquide fortement polaire est à base de l'au moins un choisi dans le groupe constitué par la monoisopropanolamine, la triéthanolamine, le propylèneglycol, la diéthanolamine, l'acide lactique, la monoéthanolamine, et le glycérol.

6. Préparation de patch selon la revendication 5, dans laquelle le composant liquide fortement polaire est à base d'acide lactique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007511605 A
- WO 2005049090 A
- EP 2759294 A

### Non-patent literature cited in the description

- *CHEMICAL ABSTRACTS,* 1643-20-5
- *CHEMICAL ABSTRACTS,* 2530-44-1
- New edition Organic Conceptual Diagram Foundation and Application. YOSHIO KOUDA, YOSHIRO SATOU, YOSHIO HONMA. SANKYO PUBLISHING, 30 November 2008